# EUROPEAN PATENT APPLICATION

(11) **EP 1 956 001 A1**
(43) Date of publication of application: **13.08.2008**
(21) Application number: 06823507.6
(22) Date of filing: 22.11.2006
(51) Int. Cl.: C07D 209/08, A61K 31/4045, A61P 13/04, A61P 13/12, A61P 21/00, A61P 25/04, A61P 43/00

(54) **PHARMACEUTICAL FOR USE IN THE TREATMENT OF URETEROLITHIASIS**

(30) Priority: 24.11.2005 JP 2005339188
(71) Applicant: Kissei Pharmaceutical Co., Ltd., Matsumoto-shi Nagano 399-8710 (JP)
(72) Inventor: KOBAYASHI, Mamoru, Azumino-shi Nagano 399-8304 (JP); TOMIYAMA, Yoshitaka, Azumino-shi Nagano 399-8304 (JP); KOBAYASHI, Kumi, Azumino-shi Nagano 399-8304 (JP)
(74) Representative: Hayes, Adrian Chetwynd
(86) International application number: PCT/JP2006/323280
(87) International publication number: WO 2007/060974

(57) **Abstract**

The present invention provides pharmaceutical compositions useful for relieving pain caused by ureteral calculi, facilitating exclusion of ureteral calculi or the like. That is, the present invention provides a pharmaceutical composition for the treatment of ureteral lithiasis, which comprises as an active ingredient an indoline derivative represented by the following general formula (I) or a salt thereof. In the formula, R represents saturated or unsaturated aliphatic acyl which may have a substituent; hydroxyalkyl; aliphatic acyloxyalkyl; lower alkyl which has as a substituent lower alkoxy, carboxy, lower alkoxycarbonyl, aryl-substituted lower alkoxycarbonyl, carbamoyl, mono or di(lower alkyl)-substituted carbamoyl or cyano; optionally substituted aromatic acyl; furoyl or pyridylcarbonyl; R¹ represents cyano or carbamoyl; and R² represents lower alkyl which may have as a substituent halogen, cyano or aryl.

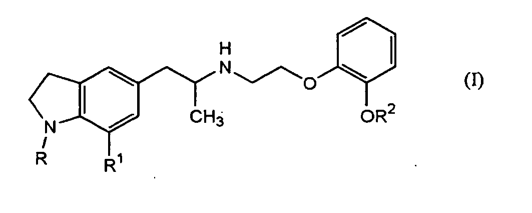

SEQUENCE LISTING

<110>  Kissei Pharmaceutical Co., Ltd.

<120>  Medicine for treatment of ureteral stone

<130>  PCT-A0633-00

<150>  JP2005/339188
<151>  2005-11-24

<160>  15

<170>  PatentIn version 3.1

<210>  1
<211>  18
<212>  DNA
<213>  Mesocricetus auratus

<400>  1
gggctactgg gcctttgg                                                    18

<210>  2
<211>  24
<212>  DNA
<213>  Mesocricetus auratus

<400>  2
ggagcatggg tatatgatag ggtt                                             24

<210>  3
<211>  19
<212>  DNA
<213>  Mesocricetus auratus

<400>  3
cgagcaactc cacactgcc                                                   19

<210>  4
<211>  21
<212>  DNA
<213>  Mesocricetus auratus

<400>  4
ctcttggcca cgatgtagac c                                                21

<210>  5
<211>  23
<212>  DNA
<213>  Mesocricetus auratus

<400>  5
gccactatgg aggttctagg ctt                                              23

<210>  6
<211>  22
<212>  DNA
<213>  Mesocricetus auratus

<400>  6
cgggttcaca cagctattga ag                                               22

<210>  7
<211>  23
<212>  DNA
<213>  Mesocricetus auratus

<400>  7
cctcaagacg gacaagtcag act                                              23

<210> 8
<211> 17
<212> DNA
<213> Mesocricetus auratus

<400> 8
cctgccggga cgttttt                                                17


<210> 9
<211> 27
<212> DNA
<213> Mesocricetus auratus

<400> 9
cgagcaagtg actcttcgta tccaccg                                     27


<210> 10
<211> 30
<212> DNA
<213> Mesocricetus auratus

<400> 10
gttccatagc tgtcaaactt tttaagttct                                  30


<210> 11
<211> 23
<212> DNA
<213> Mesocricetus auratus

<400> 11
aagatgaaca ttccgaccac aat                                         23


<210> 12
<211> 28
<212> DNA
<213> Mesocricetus auratus

<400> 12
cagggaaaag aaagcagcca aaaccttg                                    28


<210> 13
<211> 22
<212> DNA
<213> Mesocricetus auratus

<400> 13
ccttagcctc tgcaccatct ct                                          22


<210> 14
<211> 23
<212> DNA
<213> Mesocricetus auratus

<400> 14
ggtcataatg gctgggtact tga                                         23


<210> 15
<211> 19
<212> DNA
<213> Mesocricetus auratus

<400> 15
accggtacgt gggcgtgcg                                              19

## Description

### Technical Field

The present invention relates to pharmaceutical compositions for the treatment of ureteral lithiasis, which comprise as an active ingredient indoline derivatives represented by the general formula: 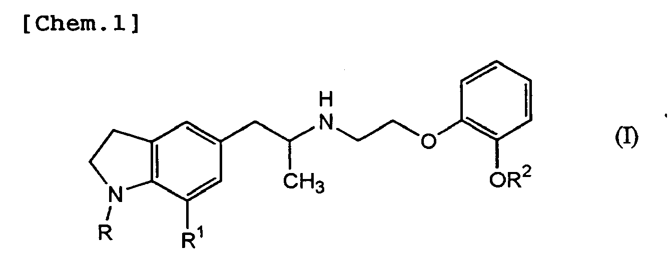 in the formula, R represents a saturated or unsaturated aliphatic acyl group which may have as a substituent one or more halogen atoms, a hydroxy group, a lower alkoxy group, a carboxy group, a lower alkoxycarbonyl group, a cycloalkyl group, or an aryl group; a hydroxyalkyl group; an aliphatic acyloxyalkyl group; a lower alkyl group which has as a substituent a lower alkoxy group, a carboxy group, a lower alkoxycarbonyl group, an aryl-substituted lower alkoxycarbonyl group, a carbamoyl group, a mono or di (lower alkyl)-substituted carbamoyl group or a cyano group; an aromatic acyl group which may have as a substituent one or more halogen atoms; a furoyl group or a pyridylcarbonyl group; R¹ represents a cyano group or a carbamoyl group; and R² represents a lower alkyl group which may have as a substituent one or more halogen atoms, a cyano group or an aryl group; or a pharmaceutically acceptable salt thereof and the like.

More particularly, the present invention relates to pharmaceutical compositions useful for relieving pain caused by ureteral calculi, facilitating exclusion of ureteral calculi, relieving hydronephrosis or reducing resistance during ureteroscope insertion and the like, which comprises as an active ingredient (-)-1-(3-hydroxypropyl)-5-((2R)-2-{[2-({2-[(2,2,2-trifluoroethyl)oxy]phenyl}oxy)ethyl]amino}propyl)-2,3-dihydro-1H-indole-7-carboxamide(generic name:silodosin) or a pharmaceutically acceptable salt thereof or the like, and the like.

### Background Art

Ureteral lithiasis means a condition in which a fallen renal stone is present in the ureter. The main symptoms are colic, hematuria, anuria, hydronephrosis and nephropyelitis (see, for example, Non-patent Reference 1).

The treatment of ureteral lithiasis includes drug therapies such as lithotriptic and analgesics in colicky attack and the like, and surgical therapies such as extracorporeal shock wave lithotripsy (ESWL), lithotripsy with an endoscope and the like. Analgesics and antispasmodics are prescribed for pain being a main symptom in ureteral lithiasis. However, the analgesic is a temporary symptomatic treatment of pain and radical treatment cannot be expected with the drug. In addition, the effect of the antispasmodics such as an anticholinergic agent is not necessarily sufficient. Therefore, an agent, which facilitates exclusion of ureteral calculi and relives pain by its potent inhibitory effect against ureteral contraction, is desired.

Concerning α₁-adrenoceptor (AR), α_{1A}, α_{1B} and α_{1D} subtypes are known. Since mRNA and protein of α_{1D}-AR are more highly expressed than those of α_{1A}- and α_{1B}-ARs in human ureteral smooth muscle, α_{1D}-AR is thought to mainly contribute to ureteral contractile function (see Non-patent Reference 2). In addition, it has been reported that α₁-AR antagonists, tamsulosin hydrochloride, terazosin and doxazosin are effective for exclusion of calculi and pain in patients with ureteral calculi. It is considered that the effects were produced by antagonism in α_{1D}-ARs (see Non-patent References 3 and 4).

α_{1A}-AR is present in the prostate, whereas α_{1D}-AR is abundantly present in the bloodvessel as well as the prostate (see Non-patent Reference 5). The two receptor subtypes participate in contractile function (see Non-patent Reference 6). In fact, it is known that tamsulosin hydrochloride decreases blood pressure more strongly than the compound represented by the above general formula (I) in anesthetized dogs (see Non-patent Reference 7). Therefore, the application of tamsulosin hydrochloride for the treatment of ureteral calculi is thought to give rise to a problem on cardiovascular systems.

It has been reported that the compound represented by the above general formula (I) or a pharmaceutically acceptable salt thereof exerts a selective α_{1A}-AR blocking effect (see Non-patent Reference 8), has an inhibitory effect against the urethral smooth muscle contraction and is useful as an agent for the treatment of dysuria accompanied by benign prostate hyperplasia and the like (for example, see Patent References 1 to 4). However, any relation between α_{1A}-AR blocking effect and ureteral calculi has not been known. In addition, it has been neither known, reported nor suggested that these compounds inhibit ureteral contraction or are useful as an agent for the treatment of ureteral lithiasis.

Patent Reference 1: Japanese Patent Publication H6-220015;
Patent Reference 2: International publication No.99-15202 pamphlet;
Patent Reference 3: International publication No.00-247998 pamphlet;
Patent Reference 4: International publication No.05-85195 pamphlet;
Non-patent Reference 1: Hyojun Hinyokikagaku (Standard urology), the 6th edition, Igakusyoin, May 15, 2002, pp. 229-237;
Non-patent Reference 2: Neurourol Urodyn, 2005, Vol. 24, pp.142-148;
Non-patent Reference 3: J. Urol. , 2003, Vol.170, pp. 2202-2205;
Non-patent Reference 4: J. Urol. , 2005, Vol.173, pp.2010-2012;
Non-patent Reference 5: J. Pharmacol. Exp. Ther., 1995, Vol.275, pp.1035-1042;
Non-patent Reference 6: Eur. J. Pharmacol. , 1996, Vol.318, pp.117-122;
Non-patent Reference 7: Int. J. Urol., 2001, Vol.8, pp.177-183;
Non-patent Reference 8: J. Pharmacol. Exp. Ther., 1999, Vol.291, pp.81-91.

### Disclosure of the Invention

### Problem to be solved by the Invention

The object of the present invention is to provide pharmaceutical compositions for the treatment of ureteral lithiasis.

### Means of solving the Problems

The present inventors have studied earnestly on the above problems, and surprisingly found that silodosin having little α_{1D}-AR blocking effect has a strong inhibitory effect against ureteral contraction and is useful for relieving pain caused by ureteral calculi, facilitating exclusion of ureteral calculi or supporting ureteroscope insertion, thereby forming the basis of the present invention.

That is, present invention relates to:
[1] a pharmaceutical composition for the treatment of ureteral lithiasis, which comprises as an active ingredient an indoline derivative represented by the general formula: 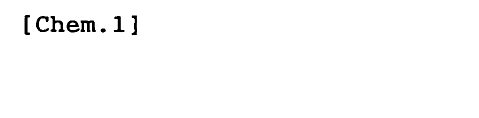 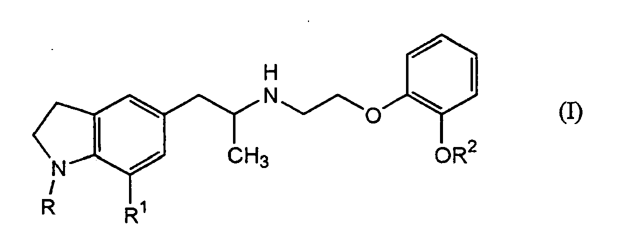 in the formula, R represents a saturated or unsaturated aliphatic acyl group which may have as a substituent one or more halogen atoms, a hydroxy group, a lower alkoxy group, a carboxy group, a lower alkoxycarbonyl group, a cycloalkyl group, or an aryl group; a hydroxyalkyl group; an aliphatic acyloxyalkyl group; a lower alkyl group which has as a substituent a lower alkoxy group, a carboxy group, a lower alkoxycarbonyl group, an aryl-substituted lower alkoxycarbonyl group, a carbamoyl group, a mono or di (lower alkyl)-substituted carbamoyl group or a cyano group; an aromatic acyl group which may have as a substituent one or more halogen atoms; a furoyl group or a pyridylcarbonyl group; R¹ represents a cyano group or a carbamoyl group; and R² represents a lower alkyl group which may have as a substituent one or more halogen atoms, a cyano group or an aryl group; or a pharmaceutically acceptable salt thereof;
[2] a pharmaceutical composition as described in the above [1] wherein the indoline derivative is silodosin;
[3] a pharmaceutical composition as described in the above [1] or [2], wherein the pharmaceutical composition is for relieving pain caused by ureteral calculi, facilitating exclusion of ureteral calculi, relieving hydronephrosis or reducing resistance during ureteroscope insertion;
[4] a pharmaceutical composition as described in any of the above [1] to [3], which is an agent for the inhibition of ureteral contraction;
[5] a method for the treatment of ureteral lithiasis, which comprises administering an effective amount of an indoline derivative represented by the above formula (I) or a pharmaceutically acceptable salt thereof;
[6] a method as described in the above [5] wherein the indoline derivative is silodosin;
[7] a use of an indoline derivative represented by the above general formula (I) or a pharmaceutically acceptable salt thereof for manufacturing a pharmaceutical composition for the treatment of ureteral lithiasis;
[8] a use as described in the above [7] wherein the indoline derivative is silodosin; and the like.

### Effect of the Invention

The pharmaceutical compositions of the present invention exert a strong inhibitory effect against ureteral contraction and are useful for the treatment of ureteral lithiasis or the like. In addition, the compounds represented by the above general formula (I) of an active ingredient of the pharmaceutical composition of the present invention are inhibitors highly selective to α_{1A}-AR compared to α_{1D}-AR (see Non-patent Reference 8), and thus, it is considered that they can be an agent for the treatment of ureteral lithiasis, which has lower cardiovascular effect.

### Brief description of Drawings

### [Figure 1]

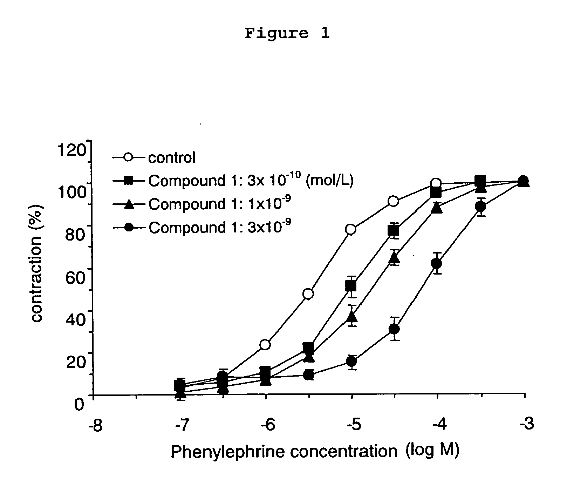

Figure 1 shows inhibitory effect against ureteral contraction. The horizontal and vertical axes show phenylephrine concentrations (log M) and ureteral contraction (%), respectively. In the figure, each curve shows the concentration response curve for phenylephrine (Control: control group) (open circle), in the presence of 3 x 10⁻¹⁰ mol/L (closed square), 1 x 10⁻⁹ mol/L (closed triangle) or 3 x 10⁻⁹ mol/L (closed circle) of Compound 1.

### Best Mode to practice the Invention

In the above general formula (I), the term "lower alkyl" means straight-chained or branched alkyl having 1 to 6 carbon atoms; the term "hydroxyalkyl" means straight-chained or branched alkyl having 2 to 6 carbon atoms and a hydroxyl group with the proviso that the hydroxyl group exists at a position other than α-position, the term "lower alkoxy" means straight-chained or branched alkoxy having 1 to 6 carbon atoms; and the term "cycloalkyl" means 5 to 7-membered cyclic alkyl, respectively. In addition, the term "aryl" means an aromatic hydrocarbon group such as phenyl, naphthyl or the like; the term "aromatic acyl" means acyl of carboxylic acid having an aryl which has the same meaning as defined above; the term "saturated or unsaturated aliphatic acyl" means acyl of straight-chained or branched alkylcarboxylic acid having 2 to 7 carbon atoms or acyl of straight-chained or branched alkenylcarboxylic acid having 3 to 7 carbon atoms; and the term "aliphatic acyloxy" means an alkylcarbonyloxyalkyl having a hydroxyl group substituted by the above aliphatic acyl group and 4 to 13 carbon atoms with the proviso that the aliphatic acyloxy group exists at a position other than α-position, respectively. Furthermore, the term "furoyl" means 2-furoyl or 3-furoyl; the term "pyridylcarbonyl" means 2-pyridylcarbonyl, 3-pyridylcarbonyl or 4-pyridylcarbonyl; and the term "halogen atom" means a fluorine atom, a chlorine atom or a bromine atom, respectively. In addition, the indoline derivatives of the general formula (I) can be prepared by the method described in Patent reference 1. As the indoline derivatives, silodosin as mentioned above, that is, (-)-1-(3-hydroxypropyl)-5-((2R)-2-{[2-({2-[(2,2,2-trifluoroethyl)oxy]phenyl}oxy)ethyl]amino}propyl)-2,3-dihydro-1H-indole-7-carboxamide (occasionally, hereinafter referred to as "Compound 1") is preferable.

As the pharmaceutically acceptable salt of the above indoline derivative, for example, in the case of a compound having a carboxy group, a salt with an inorganic base such as sodium, potassium, calcium or the like, a salt with an organic amine such as morpholine, piperidine or the like can be illustrated. In addition, among the indoline derivatives, in the case of a compound wherein the substituent R is a substituted or unsubstituted acyl group or a furoyl group, an additive salt with a monoacid such as hydrochloric acid, hydrobromic acid, sulfuric acid, methanesulfonic acid, benzenesulfonic acid, p-toluenesulfonic acid, acetic acid, citric acid, succinic acid, tartaric acid, 2,4-dimethylbenzenesulfonic acid, 2,4,6-trimethylbenzenesulfonic acid,(+)-camphorsulfonic acid, (-)-camphorsulfonic acid, 4-chlorobenzenesulfonic acid, 2-naphthalenesulfonic acid, 1-butanesulfonic acid, fumaric acid, glutamic acid, aspartic acid and the like can be illustrated. Furthermore, among the indoline derivatives, in the case of a compound wherein the substituent R is a substituted alkyl group or a pyridylcarbonyl group, an additive salt with a monoacid such as hydrochloric acid, hydrobromic acid, sulfuric acid, methanesulfonic acid, *p*-toluenesulfonic acid, 2,4-dimethylbenzenesulfonic acid, 2,5-dimethylbenzenesulfonic acid, 2,4, 6-trimethylbenzenesulfonic acid, (+)-camphorsulfonic acid, (-)-camphorsulfonic acid, 4-chlorobenzenesulfonic acid, 2-naphthalenesulfonic acid, 1-butanesulfonic acid, fumaric acid, glutamic acid, aspartic acid and the like can be illustrated.

The pharmaceutical composition of the present invention can be prepared by suitably admixing with or by diluting and dissolving with an appropriate pharmaceutical excipients such as fillers, disintegrators, binders, lubricants, diluents, buffers, isotonicities, antiseptics, moistening agents, emulsifiers, dispersing agents, stabilizing agents, dissolving aids and the like, and formulating the mixture in accordance with conventional methods.

As a dosage form of the pharmaceutical composition of the present invention, for example, a formulation for oral administration such as powders, granules, fine granules, dry syrups, tablets, capsules and the like; a formulation for parenteral administration such as injections, patches, suppositories and the like can be illustrated, and a formulation for oral administration is preferable.

The dosage of an indoline derivative represented by the above general formula (I) can be appropriately decided depending on the body weight, age, sex and degree of diseases of each patient . For example, a dosage in adult human is within the range of from 1 to 100 mg per day, preferably 1 to 50 mg per day in the case of oral administration. The dosage of silodosin in adult human is within the range of from 2 to 32 mg per day, preferably 4 to 16 mg per day in the case of oral administration. The daily dose can be divided into one to two or more doses per day and administered.

The pharmaceutical compositions of the present invention exert an inhibitory effect against ureteral contraction, and thus, are useful for the treatment of ureteral lithiasis. In the present description, ureteral lithiasis includes ureteral lithiasis diagnosed because of presenting pain, bloody urine and anuria as well as ureteral lithiasis diagnosed using ultrasonography, abdominal plain radiography, intravenous urography, CT or the like. In addition, treatment of ureteral lithiasis includes a use as an inhibitor of ureteral contraction for relieving pain caused by ureteral calculi including colic and dull pain as well, facilitating exclusion of ureteral calculi, relieving hydronephrosis or reducing resistance during ureteroscope insertion and the like.

### Examples

The present invention is further illustrated in more detail by way of the following Reference examples and Example. However, the present invention is not limited thereto.

### [Reference example 1] Quantification of mRNA expression in hamster ureter

The bilateral ureters were isolated from male Syrian hamster aged 8 weeks and RNA was extracted using ISOGEN (Nippon Gene, Toyama). Theheart andaortawere isolatedfrommale Syrian hamster aged 8 weeks and mRNAs were extracted for cDNA cloning of the three subtypes α₁ (α₁ₐ, α_{1b}, α_{1d}) - ARs.
Primers were designed on the basis of the sequence information for hamster α_{1b}-AR (J04084), rat α₁ₐ-AR (NM_017191) and rat α_{1d}-AR (NM_024483) using Primer Express Primer 2.0 (Applied Biosystems, Chiba). The prepared primers are shown as sequence No.1 and 2 (α₁ₐ), No. 3 and 4 (α_{1b}), and No. 5 and 6 (α_{1d}). cDNAs were synthesized from total RNA (1µg) in the heart and aorta, using RT reagent mixture (Two-step RT-PCR RT Reaction Mix, Applied Biosystems). Since then, cDNAs were amplified with PCR reagent mixture (AccuPrime (Registered trade mark) Taq DNA Polymerase High Fidelity, Invitrogen). Partial α₁-AR plasmids were prepared using pCRII-TOPO (Registered trade mark) and were used as standard on Real-time quantitative RT-PCR. Taq Man primers and probes for hamster α₁-AR sequences were designed using Primer Express Primer (Applied Biosystems). The prepared primers are shown as sequence No. 7 and 8 (α₁ₐ), No. 10 and 11 (α_{1b}), and No. 13 and 14 (α_{1d}), and the probes were shown as sequence No. 9 (α₁ₐ), No. 12 (α_{1b}) and No. 15 (α_{1d}). cDNAs were synthesized from total RNA with RT reagent mixture (Applied Bisosystems; Two-step RT-PCR RT Reaction Mix). The cDNAs were used as template and were measured using Taq Man Universal PCR Master mix (Applied Bisosystems). All samples were measured in duplicate.
The result is shown in Table 1. The expression of α_{1d}-AR mRNA (88.1%) was most abundant of the three α₁-AR subtypes in hamster ureter.

**[Table 1]**

| Subtype | mRNA expression level (copies/ng total RNA) | Expression rate (%) |
|---|---|---|
| α₁ₐ | 30.8±7.5 | 10.7 |
| α_{1b} | 3.5±1.3 | 1.2 |
| α_{1d} | 254.5±113.0 | 88.1 |

### [Reference example 2]

The inhibitory effects of various α₁-AR antagonists were tested using the same method as Example 1 mentioned later. Tamsulosin hydrochloride (α_{1A/1D}-AR antagonist), naftopidil (α_{1D}-AR antagonist), BMY-7378 (α_{1D}-AR antagonist) and chloroethylclonidine (α_{1B}-AR antagonist) were used. As a result, astonishingly, although compound 1 is a selective α_{1A}-AR antagonist, it exhibited high pA₂ value as shown in Table 2.

**[Table 2]**

| α₁-AR blocker | Subtype selectivity | pA₂ |
|---|---|---|
| Compound 1 | α_{1A} | 9.44 |
| tamsulosin hydrochloride | α_{1A/1D} α_{1A/1D} | 9.85 |
| naftopidil | α_{1D} | 6.54 |
| BMY-7378 | α_{1D} | 5.75 |
| chloroethylclonidine | α_{1B} | <5 |

### [Example 1]

Hamsters were killed by rapid exsanguination under ether anesthesia and both right and left ureters were isolated. The right and left ureters were removed from connective tissue in Krebs solution, and were made into tubular preparations. The ureteral preparation was suspended in organ bath containing Krebs solution maintained at 37° C and continuously gassed with a mixed gass of 95% oxygen and 5% carbon dioxide. An initial resting tension of about 0.1g was placed. The tension was isometrically measured with a force-transducer and was recorded with a polygraph. After the tension was placed, the preparation was treated with each concentration of compound 1. About 30 min later, phenylephrine (1 x 10⁻⁷ mol/L and more) was cumulatively added in 0.5-log increments and the concentration response curves were obtained. The contraction before the addition of 1 x 10⁻⁷ mol/L phenylephrine and after the addition of 1 x 10⁻³ mol/L phenylephrine were expressed as 0% and 100%, respectively. As shown in Figure 1, compound 1 from low concentrations caused concentration-dependently a parallel rightward shift of the concentration-response curve for phenylephrine in the isolated hamster ureter. That is, Compound 1 inhibited phenylephrine induced ureteral contraction in concentration dependent manner, showing that compound 1 was useful for relieving pain and facilitating exclusion of ureteral calculi and the like.

As mentioned above, it was shown that the indoline derivative represented by the above general formula (I) as typified by Compound 1 or pharmaceutically acceptable salts thereof inhibit phenylephrine-induced ureteral contraction in concentration dependent manner and are useful for relieving pain caused by ureteral calculi, facilitating exclusion of ureteral calculi or the like.

### Industrial Applicability

The pharmaceutical compositions of the present invention are extremely useful as an agent for the treatment of ureteral lithiasis and the like.

### Sequence listing free text

< Sequence No. 1> Sequence No . 1 represents the 5'-primer used in cloning of the partial sequence corresponding to hamster α₁ₐ-adrenoceptor.
<Sequence No.2> Sequence No.2 represents the 3'-primer sequence used in cloning of the partial sequence corresponding to hamster α₁ₐ-adrenalinee receptor.
<Sequence No. 3> Sequence No.3 represents the 5' -primer sequence used in cloning of the partial sequence corresponding to hamster α_{1b}-adrenoceptor.
<Sequence No.4> Sequence No. 4 represents the 3'-primer sequence used in cloning of the partial sequence corresponding to hamster α_{1b}-adrenoceptor.
<Sequence No.5> Sequence No.5 represents the 5'-primer sequence used in cloning of the partial sequence corresponding to hamster α_{1d}-adrenoceptor.
<Sequence No.6> Sequence No.6 represents the 3'-primer sequence used in cloning of the partial sequence corresponding to hamster α_{1d}-adrenoceptor.
<Sequence No.7 > Sequence No.7 represents the TaqMan (a registered trade mark) 5'-primer sequence used in real time quantitative RT-PCR to quantitate hamster α₁ₐ-adrenoceptor mRNA.
< Sequence No.8 > Sequence No.8 represents the TaqMan (a registered trade mark) 3'-primer sequence used in real time quantitative RT-PCR to quantitate hamster α₁ₐ-adrenoceptor mRNA.
<Sequence No. 9> Sequence No. 9 represents the TaqMan (a registered trade mark) probe sequence used in real time quantitative RT-PCR to quantitate hamster α₁ₐ-adrenoceptor mRNA.
<Sequence No.10> Sequence No.10 represents the TaqMan (a registered trade mark) 5'-primer sequence used in real time quantitative RT-PCR to quantitate hamster α_{1b}-adrenoceptor mRNA.
<Sequence No.11> Sequence No.11 represents the TaqMan (a registered trade mark) 3'-primer sequence used in real time quantitative RT-PCR to quantitate hamster α_{1b}-adrenoceptor mRNA.
<Sequence No.12> Sequence No.12 represents the TaqMan (a registered trade mark) probe sequence used in real time quantitative RT-PCR to quantitate hamster α_{1b}-adrenoceptor mRNA.
<Sequence No.13> Sequence No.13 represents the TaqMan (a registered trade mark) 5'-primer sequence used in real time quantitative RT-PCR to quantitate hamster α_{1d}-adrenoceptormRNA.
<Sequence No.14> Sequence No.14 represents the TaqMan (a registered trade mark) 3'-primer sequence used in real time quantitative RT-PCR to quantitate hamster α_{1d}-adrenoceptor mRNA.
<Sequence No.15> Sequence No.15 represents the TaqMan (a registered trade mark) probe sequence used in real time quantitative RT-PCR to quantitate hamster α_{1d}-adrenoceptor mRNA.

## Claims

1. A pharmaceutical composition for the treatment of ureteral lithiasis, which comprises as an active ingredient an indoline derivative represented by the general formula: 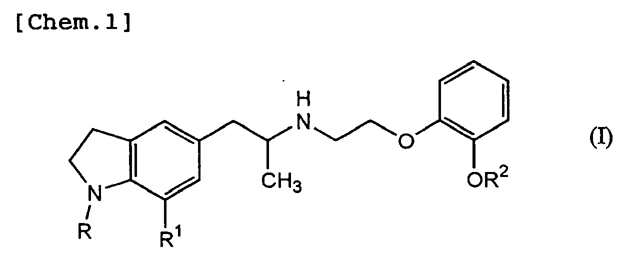 in the formula, R represents a saturated or unsaturated aliphatic acyl group which may have as a substituent one or more halogen atoms, a hydroxy group, a lower alkoxy group, a carboxy group, a lower alkoxycarbonyl group, a cycloalkyl group, or an aryl group; a hydroxyalkyl group; an aliphatic acyloxyalkyl group; a lower alkyl group which has as a substituent a lower alkoxy group, a carboxy group, a lower alkoxycarbonyl group, an aryl-substituted lower alkoxycarbonyl group, a carbamoyl group, a mono or di(lower alkyl)-substituted carbamoyl group or a cyano group; an aromatic acyl group which may have as a substituent one or more halogen atoms; a furoyl group or a pyridylcarbonyl group; R¹ represents a cyano group or a carbamoyl group; and R² represents a lower alkyl group which may have as a substituent one or more halogen atoms, a cyano group or an aryl group; or a pharmaceutically acceptable salt thereof.

2. A pharmaceutical composition as claimed in claim 1 wherein the indoline derivative is silodosin.

3. A pharmaceutical composition as claimed in claim 1 or 2, wherein the pharmaceutical composition is for relieving pain caused by ureteral calculi, facilitating exclusion of ureteral calculi, relieving hydronephrosis or reducing resistance during ureteroscope insertion.

4. A pharmaceutical composition as claimed in any of claims 1 to 3, which is an agent for the inhibition of ureteral contraction.

5. A method for the treatment of ureteral lithiasis, which comprises administering an effective amount of an indoline derivative represented by the general formula: 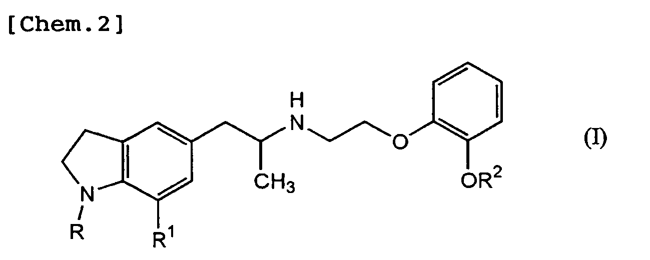 in the formula, R represents a saturated or unsaturated aliphatic acyl group which may have as a substituent one or more halogen atoms, a hydroxy group, a lower alkoxy group, a carboxy group, a lower alkoxycarbonyl group, a cycloalkyl group, or an aryl group; a hydroxyalkyl group; an aliphatic acyloxyalkyl group; a lower alkyl group which has as a substituent a lower alkoxy group, a carboxy group, a lower alkoxycarbonyl group, an aryl-substituted lower alkoxycarbonyl group, a carbamoyl group, a mono or di(lower alkyl)-substituted carbamoyl group or a cyano group; an aromatic acyl group which may have as a substituent one or more halogen atoms ; a furoyl group or a pyridylcarbonyl group; R¹ represents a cyano group or a carbamoyl group; and R² represents a lower alkyl group which may have as a substituent one or more halogen atoms, a cyano group or an aryl group; or a pharmaceutically acceptable salt thereof.

6. A method as claimed in claim 5 wherein the indoline derivative is silodosin.

7. A use of an indoline derivative represented by the general formula: 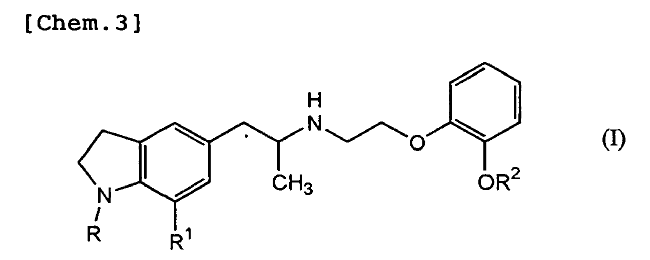 in the formula, R represents a saturated or unsaturated aliphatic acyl group which may have as a substituent one or more halogen atoms, a hydroxy group, a lower alkoxy group, a carboxy group, a lower alkoxycarbonyl group, a cycloalkyl group, or an aryl group; a hydroxyalkyl group; an aliphatic acyloxyalkyl group; a lower alkyl group which has as a substituent a lower alkoxy group, a carboxy group, a lower alkoxycarbonyl group, an aryl-substituted lower alkoxycarbonyl group, a carbamoyl group, a mono or di(lower alkyl) -substituted carbamoyl group or a cyano group; an aromatic acyl group which may have as a substituent one or more halogen atoms; a furoyl group or a pyridylcarbonyl group; R¹ represents a cyano group or a carbamoyl group; and R² represents a lower alkyl group which may have as a substituent one or more halogen atoms, a cyano group or an aryl group; or a pharmaceutically acceptable salt thereof for manufacturing a pharmaceutical composition for the treatment of ureteral lithiasis.

8. A use as claimed in claim 7 wherein the indoline derivative is silodosin.
